# FASCICULE DE BREVET EUROPEEN

(11) **EP 1 591 084 B1**
(45) Date de publication et mention de la délivrance du brevet: **10.04.2019**
(21) Numéro de dépôt: 05290890.2
(22) Date de dépôt: 21.04.2005
(51) Int. Cl.: A61F 2/40

(54) **Glenoid implant for an inverted total shoulder prothesis and prothesis therefor.**
Gelenkpfannenimplantat für eine Schultertotalprothese und Prothese hierfür.
Implant glénoidien pour prothèse totale d'épaule inversée, et prothèse totale d'épaule inversée le comprenant.

(30) Priorité: 23.04.2004 FR 0404349
(43) Date de publication de la demande: 02.11.2005
(73) Titulaire: Calamel, Serge, 13600 La Ciotat (FR); Chaudruc, Jean-Marc, 86000 Poitiers (FR); Colmar, Michel-Roland, 22000 Saint Brieuc (FR); de Cussac, Jean-Baptiste, 44500 La Baule (FR); Potaux, Frédéric, 44100 Nantes (FR); Pries, Pierre, 86170 Yversay (FR); Riguet, Jean-Michel, 86130 Jaunay Clan (FR); Wahab, Hassan, 49100 Angers (FR)
(72) Inventeur: Chaudruc Jean-Marc, 86000 Poitiers (FR); Colmar Michel-Roland, 22000 Saint Brieuc (FR); de Cussac Jean-Baptiste, 44500 La Baule (FR); Potaux Frédéric, 44100 Nantes (FR); Pries Pierre, 86170 Yversay (FR); Wahab Hassan, 49100 Angers (FR)
(74) Mandataire: Lavoix

(56) Documents cités:
- EP-A- 1 064 890
- EP-A- 1 488 764
- WO-A-01/47442
- FR-A- 2 545 352
- FR-A- 2 704 747
- US-A1- 2003 158 605
- US-B1- 6 790 234

## Description

L'invention concerne le domaine des prothèses totales d'épaule du type « prothèse inversée ».

Les prothèses totales d'épaule du type « prothèse inversée » sont utilisées lorsque la dégénérescence de l'articulation gléno-humérale du patient ne permet pas de conserver la coiffe naturelle. Elles se composent de deux implants :
- un implant huméral qui est substitué à la partie supérieure de l'humérus du patient, et comporte habituellement une quille diaphysaire, une métaphyse fixée sur la quille et un insert en polyéthylène fixé sur la métaphyse et présentant une cavité sphérique ;
- un implant glénoïdien formé habituellement d'une tête hémisphérique destinée à s'articuler dans la cavité de l'insert de l'implant huméral, et d'une pièce la supportant destinée à être ancrée dans l'omoplate du patient.

A titre d'exemple, le document EP 1 064 890 A décrit une telle prothèse totale d'épaule du type « prothèse inversée » composée de deux implants.

Un inconvénient de ces prothèses inversées est que lors des mouvements de rapprochement du bras vers le thorax et des rotations du bras visant à mettre la main contre le dos, il y a un risque que le bord de l'implant huméral ne vienne au contact du pilier de l'omoplate. En particulier, le tendon long triceps, dont le point d'ancrage est situé sur la face inférieure du pilier plus précisément (sur le tubercule sous-glénoïdien), est particulièrement vulnérable à un pincement entre l'implant huméral et le pilier. Ces contacts et ce pincement peuvent entraîner, à la longue, une inflammation du tendon et une usure du pilier.

Le but de l'invention est de proposer une configuration de l'implant glénoïdien d'une prothèse totale d'épaule inversée permettant de sauvegarder l'intégrité du tendon long triceps et du pilier de l'omoplate lors de l'utilisation de la prothèse.

A cet effet, l'invention a pour objet un implant glénoïdien pour prothèse totale d'épaule inversée, du type comportant une pièce de forme générale hémisphérique, un support destiné à être implanté dans la glène de l'omoplate du patient et comportant sur sa face avant des moyens pour lui solidariser ladite pièce hémisphérique, caractérisé en ce que ledit support comporte un bouclier prolongeant son bord inférieur et destiné à recouvrir le pilier de l'omoplate au niveau de la zone d'ancrage du tendon long triceps.

La face arrière du support peut présenter une forme convexe épousant sensiblement la forme naturelle de la glène.

Les orientations générales des faces avant et arrière du support peuvent former un angle compris entre 5 et 15°, de préférence égal à 10°, tendant à incliner la face avant vers le bas par rapport à la verticale.

Ledit implant peut conduire à une médialisation du centre de rotation de l'articulation gléno-humérale par rapport au centre naturel.

La pièce hémisphérique peut présenter un débord par rapport au bord inférieur du support.

Les moyens pour solidariser la pièce hémisphérique et le support peuvent comporter un cône morse muni d'un méplat, placé sur la face avant du support.

L'invention a également pour objet une prothèse totale d'épaule inversée, du type comportant un implant glénoïdien et un implant huméral, caractérisée en ce que l'implant glénoïdien est du type précédent.

L'angle céphalo-diaphysaire de l'implant huméral peut être compris entre 145 et 155°, préférentiellement égal à 150°.

Comme on l'aura compris, l'invention consiste à prévoir sur la pièce support de la tête hémisphérique de l'implant glénoïdien un appendice constituant un bouclier « pare-choc » qui vient s'interposer sur le trajet de l'implant huméral lors des mouvements du bras qui seraient susceptibles d'entraîner à la longue des lésions du tendon long triceps et du pilier de l'omoplate.

L'invention sera mieux comprise à la lecture de la description qui suit, donnée en référence aux figures annexées suivantes :

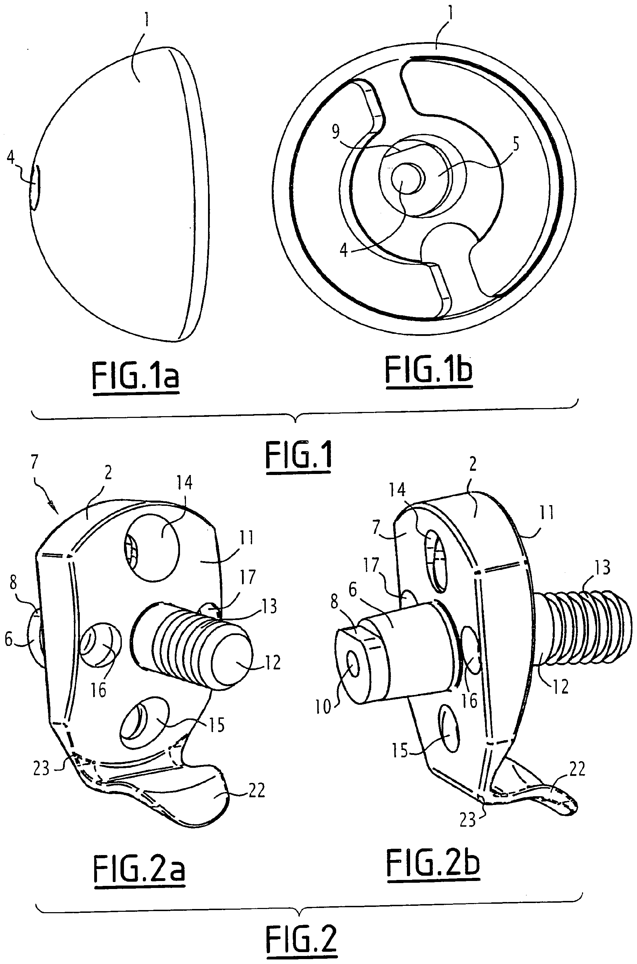

- la figure 1 qui montre un exemple de pièce hémisphérique de l'implant glénoïdien selon l'invention, vu de profil (figure 1a) et de l'arrière en perspective (fig.1b) ;
- la figure 2 qui montre un exemple de support de la pièce hémisphérique de l'implant glénoïdien selon l'invention, vu en perspective en direction de sa face arrière (fig.2a) et de sa face avant (fig.2b) ;
- la figure 3 qui montre vu de profil un exemple d'implant glénoïdien selon l'invention à l'état assemblé ;
- la figure 4 qui montre ce même exemple d'implant glénoïdien monté sur une omoplate ;
- la figure 5 qui montre un exemple de prothèse totale d'épaule inversée selon l'invention.

L'implant glénoïdien selon l'invention se compose de deux pièces : une pièce 1 de forme générale hémisphérique, et un support 2 destiné à être implanté sur l'omoplate 3 du patient, et sur lequel est fixée la pièce hémisphérique 1.

La pièce hémisphérique 1 est en un matériau tel que de l'acier inoxydable ou un autre métal biocompatible. Elle présente un orifice 4 qui la traverse de part en part pour laisser passer une vis de sécurité (non représentée) sur le support 2. Sur sa face interne, elle présente un logement 5 destiné à recevoir une excroissance du support 2, telle qu'un cône morse 6, permettant l'assemblage et la solidarisation de l'implant glénoïdien.

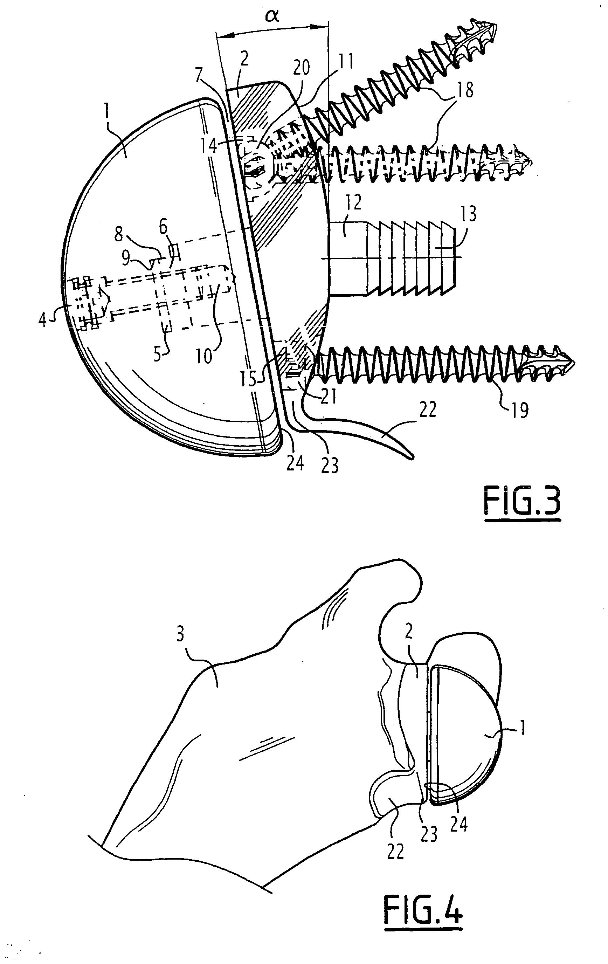

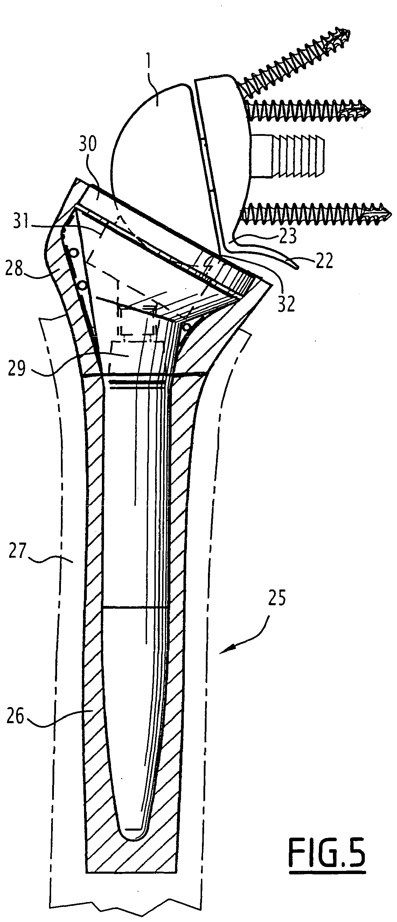

Le support 2 est également en un matériau tel que l'acier inoxydable ou un autre métal biocompatible. De manière connue, sa face avant 7 présente un cône morse 6 qui est destiné à pénétrer dans le logement 5 correspondant de la pièce hémisphérique 1. Ce cône morse 6 est terminé par une portion de section sensiblement circulaire mais présentant un méplat 8 coopérant avec un méplat 9 correspondant du logement 5. Cette configuration garantit que lors de l'utilisation de l'implant, la pièce hémisphérique 1 sera fermement empêchée de tourner autour du cône morse 6. Un orifice fileté intérieurement 10, ménagé dans le cône morse 6, reçoit la vis de sécurité de la pièce sphérique 1 sur le support 2. De manière également connue, la face arrière 11 du support 2 présente un plot 12 destiné à pénétrer dans l'omoplate 3 du patient lors de l'implantation du support. Ce plot 12 porte des barbes 13 qui empêchent sa sortie de l'omoplate 3. Le support 2 comporte également quatre orifices 14, 15, 16, 17 pour le passage de vis d'ancrages 18, 19 et la réception des têtes desdites vis 18, 19. De préférence, comme représenté, au moins certains des orifices 14-17 et des têtes 20, 21 des vis d'ancrage 18, 19 ont des configurations qui rendent possible un choix par le chirurgien de l'orientation de la pénétration des vis d'ancrage 18, 19 dans l'omoplate 3 : en particulier des surfaces de contact sphériques entre les têtes 20, 21 et le fond de l'orifice 14-17 où elles sont logées, et un diamètre de l'orifice 14-17 au niveau de la face arrière du support 11 très supérieur au diamètre de la vis 18, 19 qui le traverse. Les figures 3 et 5 montrent en superposition deux inclinaisons possibles pour l'une 18 des vis d'ancrage.

La particularité du support 2 de l'implant glénoïdien selon l'invention est de comporter un bouclier 22 qui prolonge le bord inférieur 23 du support 2. Il est destiné à venir recouvrir le pilier de l'omoplate 3, notamment au niveau de la zone d'ancrage du tendon long triceps (tubercule sous-glénoïdien). De cette façon, on évite que lors des mouvements de rapprochement du bras vers le thorax et des rotations main dans le dos, l'implant huméral ne vienne heurter l'omoplate 3 ou le tendon long triceps, avec les risques de lésions associées si ces contacts se répètent. On voit sur la figure 5 que le bord supérieur de l'implant huméral se trouve effectivement à proximité de la zone concernée du pilier de l'omoplate 3. En général, un contact entre le bouclier 22 et l'omoplate n'a lieu que lors de certains mouvements du patient.

L'exemple d'implant glénoïdien selon l'invention représenté sur les figures comporte également d'autres caractéristiques particulières, qui ne sont cependant pas obligatoires pour la mise en oeuvre de l'invention. Elles sont utilisables indépendamment les unes des autres, et pourraient également être utilisées sur des implants glénoïdiens classiques pour prothèse totale d'épaule inversée, donc dépourvus du bouclier 22.

La face arrière 11 du support 3, donc celle qui vient au contact de la glène, présente un ancrage convexe anatomique, c'est-à-dire épousant sensiblement la forme naturelle de la glène. On minimise ainsi la perte de substance osseuse lors de la préparation de la glène. La surface de la face arrière 11 est recouverte de titane poreux et d'hydroxyapatite, sauf sur le plot 12 où il n'y a que de l'hydroxyapatite. On vise ainsi à favoriser la croissance osseuse à l'interface support 3-glène.

Les orientations générales des faces avant 7 et arrière 11 du support 3 ne sont pas parallèles, mais forment entre elles un angle α de l'ordre de 5 à 15°, typiquement 10°, tendant à incliner la face avant 7 vers le bas par rapport à la verticale. Par ailleurs, le centre de rotation de l'articulation gléno-humérale ainsi reconstituée est médialisé par rapport au centre naturel. On permet ainsi le démarrage de l'épaule en abduction et dans le plan de l'omoplate, grâce à l'optimisation de la force du deltoïde que procure cette configuration.

Enfin, un autre facteur permettant d'éviter les contacts entre l'implant huméral et l'omoplate ou le tendon long triceps est le léger débord inférieur 24 que présente la pièce hémisphérique 1 par rapport à la face avant 7 du support 2, et qui contribue à maintenir une distance adéquate entre l'implant huméral et l'omoplate (voir figure 5).

La figure 5 représente une prothèse totale d'épaule inversée à laquelle est intégré l'implant glénoïdien selon l'invention. L'implant huméral 25 est classiquement composé de trois parties :
- une tige diaphysaire 26 à insérer dans l'humérus 27 amputé de sa partie supérieure ; elle peut être cimentée à l'intérieur de l'humérus 27, ou y être fixée par simple croissance osseuse, auquel cas un revêtement d'hydroxyapatite est de préférence appliqué sur la tige 26 ;
- une métaphyse 28 qui coiffe la tige diaphysaire 26 et lui est fixée par un cône morse 29, et est elle aussi revêtue ou non d'hydroxyapatite ;
- un insert 30 en polyéthylène qui est reçu dans un évidement 31 de la métaphyse 28, et qui comporte sur sa face supérieure une cavité 32 sphérique de courbure correspondant à celle de la pièce hémisphérique 1.

Une vis centrale de compression (non représentée) assure la solidarisation de ces trois parties.

L'angle céphalo-diaphysaire de l'implant huméral 25 est de préférence de l'ordre de 145 à 155°, typiquement 150°. On obtient ainsi une légère varisation de l'épiphyse qui permet, là encore, d'optimiser la prévention du contact entre la métaphyse 28 et le pilier de l'omoplate 3.

Les dimensions des différentes pièces peuvent varier en fonction de l'anatomie des patients auxquels elles sont destinées. Typiquement, on peut prévoir trois tailles différentes pour les composants de l'implant glénoïdien et la métaphyse, et cinq tailles différentes pour la tige diaphysaire. La possibilité de choisir la taille de l'implant glénoïdien et l'orientation des vis d'ancrage 18, 19 permet d'optimiser l'ancrage des vis 18, 19 en le réalisant dans un point capital de solidité osseuse de l'omoplate, à savoir la base de la coracoïde. Cela facilite l'utilisation de l'implant glénoïdien selon l'invention, en particulier en tant qu'implant de reprise, destiné à remplacer une prothèse défaillante.

Bien entendu, des variantes d'exécution de l'invention s'écartant de l'exemple décrit et représenté sont possibles. Par exemple, le cône morse 6 pourvu d'un méplat 8, permettant la fixation de la pièce hémisphérique 1 sur le support 2, peut être remplacé par tout autre moyen de fixation ferme de ces deux pièces l'une à l'autre, interdisant leur déplacement relatif. La pièce hémisphérique 1 devrait alors être modifiée en conséquence. L'implant huméral 25 peut être de tout type connu compatible avec l'implant glénoïdien selon l'invention. Les matériaux qui ont été cités à propos des différents constituants peuvent également être modifiés : par exemple la pièce hémisphérique 1 pourrait être non en métal mais en céramique.

## Revendications

1. Implant glénoïdien pour prothèse totale d'épaule inversée, du type comportant une pièce (1) de forme générale hémisphérique, un support (2) destiné à être implanté dans la glène de l'omoplate (3) du patient et comportant sur sa face avant (7) des moyens pour lui solidariser ladite pièce hémisphérique (1), **caractérisé en ce que** ledit support (2) comporte un bouclier (22) prolongeant son bord inférieur (23) et destiné à recouvrir le pilier de l'omoplate (3) au niveau de la zone d'ancrage du tendon long triceps.

2. Implant glénoïdien selon la revendication 1, **caractérisé en ce que** la face arrière (11) du support (2) présente une forme convexe épousant sensiblement la forme naturelle de la glène.

3. Implant glénoïdien selon la revendication 1 ou 2, **caractérisé en ce que** les orientations générales des faces avant (7) et arrière (11) du support (2) forment un angle (α) compris entre 5 et 15°, de préférence égal à 10°, tendant à incliner la face avant (7) vers le bas par rapport à la verticale.

4. Implant glénoïdien selon l'une des revendications 1 à 3, **caractérisé en ce qu'**il conduit à une médialisation du centre de rotation de l'articulation gléno-humérale par rapport au centre naturel.

5. Implant glénoïdien selon l'une des revendications 1 à 4, **caractérisé en ce que** la pièce hémisphérique (1) présente un débord (24) par rapport au bord inférieur (23) du support (2).

6. Implant glénoïdien selon l'une des revendications 1 à 5, **caractérisé en ce que** les moyens pour solidariser la pièce hémisphérique (1) et le support (2) comportent un cône morse (6) muni d'un méplat (8), placé sur la face avant (7) du support (2).

7. Prothèse totale d'épaule inversée, du type comportant un implant glénoïdien et un implant huméral (25), **caractérisée en ce que** l'implant glénoïdien est du type selon l'une des revendications 1 à 6.

8. Prothèse totale d'épaule inversée selon la revendication 7, **caractérisée en ce que** l'angle céphalo-diaphysaire de l'implant huméral (25) est compris entre 145 et 155°, préférentiellement égal à 150°.

## Patentansprüche

1. Glenoidales Implantat für eine inverse Gesamt-Schulterprothese, des Typs aufweisend ein im Allgemeinen halbkugelförmiges Teil (1), einen Halter (2), der dazu bestimmt ist, in die Gelenkpfanne des Schulterblatts (3) des Patienten implantiert zu werden, und der auf seiner Vorderseite (7) Mittel aufweist, um ihn an dem besagten halbkugelförmigen Teil (1) zu befestigen, **dadurch gekennzeichnet, dass** der besagte Halter (2) einen Schild (22) aufweist, der seinen unteren Rand (23) verlängert und der dazu bestimmt ist, die Säule des Schulterblatts (3) auf Niveau der Verankerungszone der langen Trizeps-Sehne abzudecken.

2. Glenoidales Implantat gemäß Anspruch 1, **dadurch gekennzeichnet, dass** die Hinterseite (11) des Halters (2) eine konvexe Form hat, die im Wesentlichen der natürlichen Form der Gelenkpfanne angepasst ist.

3. Glenoidales Implantat gemäß Anspruch 1 oder 2, **dadurch gekennzeichnet, dass** die allgemeinen Ausrichtungen der Vorderseite (7) und der Hinterseite (11) des Halters (2) einen Winkel (α) bilden, der zwischen 5 und 15° liegt, bevorzugt gleich 10° ist, dazu tendierend, die Vorderseite (7) bezüglich der Vertikalen nach unten zu neigen.

4. Glenoidales Implantat gemäß einem der Ansprüche 1 bis 3, **dadurch gekennzeichnet, dass** es eine Medialisierung des Drehzentrums des Glenohumeralgelenks bezüglich des natürlichen Zentrums durchführt.

5. Glenoidales Implantat gemäß einem der Ansprüche 1 bis 4, **dadurch gekennzeichnet, dass** das halbkugelförmige Teil (1) bezüglich des unteren Rands (23) des Halters (2) einen Sims (24) darstellt.

6. Glenoidales Implantat gemäß einem der Ansprüche 1 bis 5, **dadurch gekennzeichnet, dass** die Mittel zum Aneinander-Befestigen des halbkugelförmigen Teils (1) und des Halters (2) einen Morsekegel (6) mit einer Flanke (8) aufweisen, der auf der Vorderseite (7) des Halters (2) angeordnet ist.

7. Inverse Gesamt-Schulterprothese, des Typs aufweisend ein glenoidales Implantat und ein humerales Implantat (25), **dadurch gekennzeichnet, dass** das glenoidale Implantat von Typ gemäß einem der Ansprüche 1 bis 6 ist.

8. Inverse Gesamt-Schulterprothese gemäß Anspruch 7, **dadurch gekennzeichnet, dass** der cephal-diaphysäre Winkel des humeralen Implantats (25) zwischen 145 und 155° liegt, bevorzugt gleich 150° ist.

## Claims

1. Glenoid implant for reverse total shoulder prosthesis, of the type comprising a part (1) of generally hemispherical shape, a support (2) which is to be implanted in the glenoid of the scapula (3) of the patient and which has on its front face (7) means for securing said hemispherical part (1) thereto, **characterised in that** said support (2) comprises a shield (22) which extends its lower edge (23) and is to cover the pillar of the scapula (3) in the region of the anchoring zone of the long triceps tendon.

2. Glenoid implant according to claim 1, **characterised in that** the rear face (11) of the support (2) has a convex shape which substantially matches the natural shape of the glenoid.

3. Glenoid implant according to claim 1 or 2, **characterised in that** the general orientations of the front (7) and rear (11) faces of the support (2) form an angle (α) of between 5 and 15°, preferably equal to 10°, tending to incline the front face (7) downwards relative to the vertical.

4. Glenoid implant according to any one of claims 1 to 3, **characterised in that** it leads to medialisation of the centre of rotation of the glenohumeral joint relative to the natural centre.

5. Glenoid implant according to any one of claims 1 to 4, **characterised in that** the hemispherical part (1) has an overhang (24) relative to the lower edge (23) of the support (2).

6. Glenoid implant according to any one of claims 1 to 5, **characterised in that** the means for securing the hemispherical part (1) and the support (2) comprise a Morse taper (6) provided with a flat portion (8), placed on the front face (7) of the support (2).

7. Reverse total shoulder prosthesis, of the type comprising a glenoid implant and a humeral implant (25), **characterised in that** the glenoid implant is of the type according to any one of claims 1 to 6.

8. Reverse total shoulder prosthesis according to claim 7, **characterised in that** the cephalodiaphyseal angle of the humeral implant (25) is between 145 and 155°, preferably equal to 150°.
